# EUROPÄISCHE PATENTSCHRIFT

(11) **EP 0 476 242 B1**
(45) Veröffentlichungstag und Bekanntmachung des Hinweises auf die Patenterteilung: **10.01.1996**
(21) Anmeldenummer: 91110542.7
(22) Anmeldetag: 26.06.1991
(51) Int. Cl.: A61B 5/07

(54) **Verfahren zur Überwachung eines Patienten im Hinblick auf Abstossungsreaktionen eines implantierten Herzens**
Method of monitoring a patient with a view to rejection response of an implanted heart
Procédé pour surveiller un patient dans le cadre d'une réaction de rejet d'un coeur transplanté

(30) Priorität: 21.09.1990 DE 4029961
(43) Veröffentlichungstag der Anmeldung: 25.03.1992
(73) Patentinhaber: Fehling, Guido, D-63791 Karlstein (DE)
(72) Erfinder: Müller, Johannes, Dipl.-Ing. Dr. med., W-1000 Berlin 31 (DE)
(74) Vertreter: Patentanwälte Westphal, Buchner, Mussgnug Neunert, Göhring

(56) Entgegenhaltungen:
- FR-A- 2 193 320
- FR-A- 2 290 874
- IEEE TRANSACTION ON BIOMEDICAL ENGINEERING BME-33, Nr. 2, Februar 1986, Seiten 215-221, New-York, US; P.R. TROYK et al.: "Design and Implementation of an implantable Goniometer"
- idem

## Beschreibung

Die Erfindung betrifft ein Verfahren zur Überwachung eines Patienten im Hinblick auf Abstoßungsreaktionen eines implantierten Herzens gemäß dem Oberbegriff des Patentanspruchs 1.

Bei der Herztransplantation stellt die Abstoßung des transplantierten Herzens ein wesentliches Problem dar. Es ist daher wichtig, eine beginnende Abstoßungsreaktion möglichst frühzeitig zu diagnostizieren, um rechtzeitig eine immunsuppressive Therapie einleiten zu können. Zur Überwachung des Patienten auf beginnende Abstoßungsreaktionen des implantierten Herzens stehen verschiedene Methoden zur Verfügung.

Bei der Endomyokardbiopse werden dem transplantierten Herzen Gewebsproben entnommen und histologisch untersucht. Die invasive Entnahme von Gewebsproben kann nur in spezialisierten Klinikzentren durchgeführt werden. Die Untersuchung kann nicht beliebig häufig wiederholt werden, so daß das Risiko besteht, daß im zeitlichen Intervall zwischen zwei Biopsie-Eingriffen eine Abstoßungsreaktion beginnt und sich zu einer klinisch bedrohlichen Abstoßungskrise entwickelt. Weiter ist das Verfahren nur mäßig repräsentativ, da jeweils nur einige wenige Gewebsproben entnommen werden können, die nicht notwendigerweise eine bereits beginnende Abstoßungsreaktion nachweisen.

Weiter ist eine elektrophysiologische Methode bekannt, bei welcher das intramyokardiale Elektrogramm gemessen und dessen Veränderung zur Diagnose der Abstoßungsreaktion verwendet wird. Die Diagnose mittels des intramyokardialen Elektrogrammes ist mit gewissen Unsicherheiten verbunden, da die Meßergebnisse auch durch andere Einflüsse wie die tagesrhythmischen Schwankungen, den Belastungszustand des Patienten und die Medikation verändert werden können.

Schließlich ist eine Überwachung der Herzfunktion durch die Echokardiographie bekannt. Dieses Verfahren, das die Bewegung der Herzwand durch Ultraschallecho bestimmt, kann ebenfalls nur in spezialisierten Kliniken von Fachpersonal durchgeführt werden. Die zeitlichen Untersuchungsintervalle sind daher zwangsläufig relativ groß und die Untersuchung ist kostenaufwendig. Das Untersuchungsverfahren kann außerdem z. B. durch Emphyseme oder Adipositas des Patienten erschwert oder sogar unmöglich gemacht werden.

Eine Überwachung eines Patienten auf Abstoßungsreaktionen eines implantierten Herzens mit den Merkmalen des Oberbegriffs des Patentanspruchs 1 ist aus der US 4 237 900 zu entnehmen. Dort wird der Druck im Herzen überwacht.

Aus IEEE Transactions on Biomedical Engineering, vol. BME-33, No.2, 1986; Seiten 215 bis 221 ist es bekannt, die Winkelbewegung von Gelenken dadurch zu bestimmen, daß Permanentmagnete in den einen Teil des Gelenkes und ein Hall-Sensor in den anderen Teil des Gelenkes eingesetzt werden. Die gegenseitige Bewegung der beiden Gelenkteile ergibt ein Signal des Hall-Sensors, welches die Bewegung des Gelenkes repräsentiert. Bei einer Implantation ist eine induktive Übertragung der Meßsignale mittels elektromagnetischer Trägerwellen aus dem Körper zu einem extrakorporalen Gerät vorgesehen.

Der Erfindung liegt die Aufgabe zugrunde, ein Verfahren der eingangs genannten Art zur Verfügung zu stellen, das zur kostengünstigen, kontinuierlichen Überwachung der Funktion eines transplantierten Herzens, vor allem die Erfassung der Bewegung der Herzwand, ermöglicht.

Diese Aufgabe wird mit einem Verfahren der eingangs genannten Gattung erfindungsgemäß gelöst durch die Merkmale des kennzeichnenden Teils des Patentanspruchs 1.

Vorteilhafte Ausführungsformen der Erfindung sind in den Unteransprüchen angegeben.

Das erfindungsgemäße Verfahren nützt die funktionelle Bewegung der Herzwand zur Ermittlung elektrischer Signale aus, die eine Diagnostik beginnender Abstoßungsreaktionen ermöglichen. Die Herzwand, am stärksten die linksventrikuläre Herzwand, ändert ihre Dicke wechselnd zwischen Systole und Diastole. Dies entspricht einer Abstandsänderung zwischen Endokard und Epikard. Außerdem tritt eine gewisse Rotationsbewegung zwischen Endokard und Epikard auf. Diese Bewegungen der Herzwand werden durch Abstoßungsreaktionen beeinflußt.

Erfindungsgemäß wird eine Sensoreinheit verwendet, die in die Wand des transplantierten Herzens, vorzugsweise in die linksventrikuläre Herzwand, implantiert ist, die aus einem Permanentmagneten und einem magnetisch aktiven Halbleiterelement besteht. Der Permanentmagnet wird bevorzugt in das Epikard implantiert, während das Halbleiterelement bevorzugt in das Endokard implantiert wird. Die Bewegung der Herzwand führt zu einer Abstandsänderung zwischen dem Permanentmagneten und dem magnetisch aktiven Halbleiterelement. Dadurch ändert sich die auf das Halbleiterlement einwirkende magnetische Feldstärke des Permanentmagneten, so daß von dem Halbleiterelement ein von der Herzwandbewegung abhängiges Signal erhalten werden kann. Die Auswertung dieses Signals läßt die Diagnose einer beginnenden Abstoßungsreaktion zu.

Da die Herzwandbewegung durch die Sensoreinheit unmittelbar an der Herzwand gemessen wird, kann die Messung nicht durch außerhalb des Herzens liegende Einflüsse, wie Emphyseme oder Adipositas beeinträchtigt werden.

Weiter ist eine kontinuierliche Überwachung bzw. eine Überwachung in kurzen Zeitabständen möglich, wenn die Meßwerte der Sensoreinheit in einem extrakorporalen Steuer- und Registriergerät gespeichert werden. Der Patient muß sich zur Überwachung nicht jedes Mal in die Klinik begeben, es ist vielmehr nur erforderlich, die in vorgegebenen Abständen aufgezeichneten Meßwerte zur Auswertung in die Klinik zu übermitteln. Dies kann vorzugsweise z.B. durch telefonische Datenübertragung geschehen.

Besonders zweckmäßig ist es, wenn die Sensoreinheit durch ein ebenfalls implantierbares Bauteil elektrisch versorgt wird, das auch die Meßwerte aufnimmt und über eine Sender- und Empfängerschaltung mittels elektromagnetischer Wellen an das extrakorporale Steuer- und Registriergerät überträgt. Der Patient kann sich dann praktisch ungehindert bewegen. Er muß sich nur in den vorgegebenen regelmäßigen Zeitabständen, beispielsweise während der Nachtruhe, die Antennenspule des extrakorporalen Steuer- und Registriergerätes am Körper über dem Herzen anordnen. Das Steuer- und Registriergerät sendet ein Einschaltsignal an das implantierte Bauteil, um den Meßvorgang auszulösen. Die Meßwerte werden von dem Bauteil an das Steuer- und Registriergerät übertragen und in diesem gespeichert. Zu einem von der Messung unabhängigen späteren Zeitpunkt können die in dem Steuer- und Registriergerät gespeicherten Meßwerte z.B. über eine telefonische Datenleitung an die Klinik übertragen und dort ausgewertet werden.

Der Patient muß sich daher nur dann in die Klinik begeben, wenn diese Auswertung zu Ergebnissen führt, die eine eingehendere Untersuchung notwendig machen.

Da es bei der Messung der Herzwandbewegung auf die Relativbewegung zwischen dem Permanentmagneten und dem magnetisch aktiven Halbleiterelement ankommt, kann selbstverständlich auch der Permanentmagnet in das Endokard und das Halbleiterelement entsprechend in das Epikard implantiert werden.

Das magnetisch aktive Halbleiterelement ist vorzugsweise ein Hall-Generator. Dieser besteht aus einem Halbleiterplättchen, das durch das implantierte Bauteil gespeist von einem Strom konstanter Stärke durchflossen wird. Senkrecht zu der Richtung des durchfließenden Stromes wird als Meßsignal die Hall-Spannung abgegriffen.

Anstelle eines Hall-Generators kann als Halbleiterelement auch eine Feldplatte verwendet werden, die einen durch die magnetische Feldstärke gesteuerten Halbleiterwiderstand darstellt. Ebenso kann als Halbleiterelement auch eine Magnetdiode eingesetzt werden, die ihren Innenwiderstand in Abhängigkeit des Magnetfeldes ändert.

Es wird bevorzugt wenn während der Herztransplantations-Operation in die linksventrikuläre Herzwand endokardial ein Hall-Generator und epikardial ein kleiner Permanentmagnet implantiert wird. Zusätzlich wird herznah ein elektronisches Bauteil implantiert, welches über hochflexible Verbindungskabel mit dem Hall-Generator verbunden ist. Das Bauteil enthält eine Batterie als Stromquelle für den Hall-Generator und zur elektrischen Versorgung der elektronischen Schaltungen des Bauteils. Weiter enthält das Bauteil eine elektronische Schaltung zur Messung der Hall-Spannung des Hall-Generators. Schließlich enthält das Bauteil eine Sender- und Empfängerschaltung, die vorzugsweise mit einer Trägerfrequenz von z.B. 40 kHz arbeitet. Die Sender- und Empfängerschaltung ist über die elektromagnetischen Trägerwellen induktiv mit einer Antennenspule eines extrakorporalen Steuer- und Registriergerätes koppelbar, wenn diese Antennenspule über dem implantierten Herzen außen auf dem Körper des Patienten angeordnet wird.

Zur Durchführung des Überwachungsvorgangs sendet das Steuer- und Registriergerät ein Einschaltsignal an die Sende- und Empfängerschaltung, um das Bauteil einzuschalten. Es wird dann ein Strom durch den Hall-Generator geleitet und die sich in Abhängigkeit der Herzwandbewegung ändernde Hall-Spannung des Hall-Generators gemessen. Die Meßwerte werden von der Sende- und Empfängerschaltung aus dem Körper des Patienten herausgesendet, durch die Antennenspule aufgenommen und in dem Steuer- und Registriergerät gespeichert. Nach Ablauf des in dem Steuer- und Registriergerät programmierten Meßzyklus wird das Bauteil durch ein von dem Steuer- und Registriergerät ausgesandtes Abschaltsignal wieder abgeschaltet, damit die Batterie des Bauteils möglichst wenig belastet wird. Die in dem Steuer- und Registriergerät gespeicherten Meßwerte können über ein Modem und eine telefonische Datenfernübertragung von der Klinik zu einem geeigneten Zeitpunkt zur Auswertung abgerufen werden.

## Patentansprüche

1. Verfahren zur Überwachung eines Patienten im Hinblick auf Abstoßungsreaktionen eines implantierten Herzens mittels einer in den Körper des Patienten implantierbaren elektronischen Sensoreinheit und eines implantierbaren Bauteiles zur elektrischen Versorgung der Sensoreinheit und zur Übertragung der Meßwerte der Sensoreinheit aus dem Körper des Patienten zu einem extrakorporalen Steuer- und Registriergerät, dadurch gekennzeichnet, daß ein in das Epikard oder in das Endokard implantierter Permanentmagnet der Sensoreinheit in einem entsprechend in das Endokard oder in das Epikard implantierten, durch das Bauteil elektrisch versorgten magnetisch aktiven Halbleiterelement der Sensoreinheit der durch die Bewegung der Herzwand verursachten Abstandsänderung zwischen dem Permanentmagneten und dem Halbleiterelement entsprechende elektrische Signale erzeugt, die durch das Bauteil aus dem Körper des Patienten übertragen werden.

2. Verfahren nach Anspruch 1, dadurch gekennzeichnet, daß das implantierbare Bauteil mittels elektromagnetischer Wellen induktiv mit dem extrakorporalen Steuer- und Registriergerät gekoppelt wird.

3. Verfahren nach Anspruch 1 oder 2, dadurch gekennzeichnet, daß als Halbleiterelement ein Hall-Generator, eine Feldplatte oder eine Magnetdiode verwendet wird.

## Claims

1. Method of monitoring a patient in relation to rejection reactions of an implanted heart by means of an electronic sensor unit implantable in the body of the patient and an implantable component for the electrical supply of the sensor unit and for the transmission of the measured values of the sensor unit from the body of the patient to an extracorporeal control and recording instrument, characterized in that a permanent magnet of the sensor unit, which permanent magnet is implanted in the epicardium or in the endocardium, generates electrical signals in a magnetically active semiconductor element of the sensor unit, which magnetically active semiconductor element is correspondingly implanted in the endocardium or in the epicardium and is electrically supplied by the component, which electrical signals correspond to the change in distance, caused by the movement of the heart wall, between the permanent magnet and the semiconductor element and are transmitted by the component from the body of the patient.

2. Method according to Claim 1, characterized in that the implantable component is inductively coupled to the extracorporeal control and recording instrument by means of electromagnetic waves.

3. Method according to Claim 1 or 2, characterized in that a Hall generator, a field plate or a magnetic diode is used as semiconductor element.

## Revendications

1. Procédé de surveillance des réactions de rejet d'un coeur implanté dans un patient, à l'aide d'un capteur électronique implanté dans le corps du patient et d'un composant implanté, pour assurer l'alimentation électrique du capteur et pour transmettre les valeurs de mesure du capteur hors du corps du patient vers un appareil de commande et d'enregistrement situé à l'extérieur du corps, procédé caractérisé en ce qu'un aimant permanent appartenant au capteur et qui est implanté dans l'épicarde ou dans l'endocarde, crée dans un élément semi-conducteur magnétiquement actif faisant partie du capteur, alimenté électriquement par le composant et qui est implanté de façon correspondante dans l'endocarde ou l'épicarde, crée des signaux électriques correspondant aux variations entre distance entre l'aimant permanent et l'élément semi-conducteur, variations provoquées par le mouvement de la cloison cardiaque, et ces signaux sont transmis par le composant hors du corps du patient.

2. Procédé selon la revendication 1, caractérisé en ce que le composant implanté est couplé par des ondes électromagnétiques, de façon inductive à l'appareil de commande et d'enregistrement situé à l'extérieur du corps.

3. Procédé selon la revendication 1 ou 2, caractérisé en ce que l'élément semi-conducteur est un élément à effet Hall, une plaque de champ ou une diode magnétique.
